# EUROPEAN PATENT SPECIFICATION

(11) **EP 3 677 214 B1**
(45) Date of publication and mention of the grant of the patent: **18.11.2020**
(21) Application number: 19425001.5
(22) Date of filing: 02.01.2019
(51) Int. Cl.: A61B 90/70

(54) **TROLLEY FOR WASHING AND DISINFECTING ROBOTIC SURGICAL INSTRUMENTS**
AUFNAHMEWAGEN ZUM WASCHEN UND DESINFEKTIEREN CHIRURGISCHER ROBOTERINSTRUMENTE
CHARIOT RÉCEPTEUR POUR LAVAGE ET DÉSINFECTER DES INSTRUMENTS CHIRURGICAUX ROBOTISÉS

(43) Date of publication of application: 08.07.2020
(73) Proprietor: Bonferraro S.p.A., 37060 Bonferraro (VR) (IT)
(72) Inventor: Gobbi, Ezio, 46037 Roncoferraro MN (IT); Benfatti, Oscar, 37063 Isola della Scala VR (IT); Tosi, Mauro, 37051 Bovolone VR (IT)
(74) Representative: Concone, Emanuele

(56) References cited:
- DE-A1-102014 111 319
- US-A1- 2016 175 062
- US-A1- 2016 242 868
- "Miele Professional A502", , 1 November 2015 (2015-11-01), XP002791628, Retrieved from the Internet: URL:www.miel-professional.com [retrieved on 2019-05-27]

## Description

This invention concerns apparatuses for the washing and disinfection (thermodisinfectors) of robotic surgical instruments, and in particular a trolley used to load into a thermodisinfector the instruments to be washed and provided with an integrated system for locking the instruments and the nozzles that supply the wash/rinse fluid.

It is known that a trolley for the washing and disinfection of robotic surgical instruments (e.g. the Da Vinci series instruments from Intuitive Surgical, Inc. of Sunnyvale, California, US) traditionally includes one or more horizontal manifolds provided with flexible washing tubes that end with nozzles to be inserted into the instruments, said nozzles being typically arranged in pairs since each instrument has a primary irrigation port and a secondary irrigation port. The trolley also includes a support and locking system for these instruments and the relevant nozzles, to ensure that they do not move during the wash cycle and that the washing is performed correctly.

In prior art trolleys known to the applicant, the locking system is designed to act on each instrument individually or on all instruments connected to a manifold at the same time. It is clear that individual locking implies a much more complex and expensive system than multiple locking, and also the labour and time required for its use are obviously greater as the instruments must be locked and unlocked one by one.

Instead, in a trolley by Miele & Cie. KG (Gütersloh, DE), marketed as model A 502, also disclosed in DE 102014111319 A1, the simultaneous locking of all instruments is achieved by means of a locking unit arranged transversely to them and rotating around an axis of rotation, and also provided with an axial locking/unlocking stroke along that axis. More specifically, said locking unit extends parallel to the manifold between two seats located at the ends of the instrument support, one of these seats being provided with two retaining notches that extend in the axial direction and are formed in two plates that are adjacent and orthogonal to each other, one plate being on the upper side of the seat and the other plate being arranged on the side facing the inside of the trolley.

The locking unit has essentially an L shape in lateral view, being composed of a straight rod and two slightly shorter rods parallel thereto that are fixed on the straight rod by means of curved terminal portions, said shorter rods defining respectively with the straight rod two planes that are perpendicular to each other. The straight rod is rotatably mounted in the seats to act as an axis of rotation, in the angle of the L shape, and can also slide axially in these seats overcoming the resistance of two end springs that bias it towards the retaining notches.

Of the two shorter rods, a first rod serves as a maneuvering rod to control the rotation of the locking unit and the second rod serves as an engagement rod to lock the instruments and the washing nozzles. For this purpose, each pair of nozzles is inserted into a pair of holes formed in the short side of an L-shaped bracket, the long side of which rests on the top of a surgical instrument and ends with a semicircular seat facing outwards from the L-shaped form.

In practice, the instruments are placed in their seats on the trolley support and the nozzles are inserted through the brackets and then into the irrigation ports of the instruments, so that the long side of each L-shaped bracket rests on top of the relevant instrument. To perform the locking, the operator grips the maneuvering rod and slides the locking unit axially overcoming the resistance of the end springs to disengage the curved end of the engagement rod from the upper retaining notch, then rotates the locking unit 90° inward so that the engagement rod engages in the semicircular seats at the ends of the brackets, and finally releases the maneuvering rod so that the end springs push the engagement rod to engage the internal retaining notch so as to lock the brackets and thus both the instruments and the washing nozzles (the unlocking is obviously obtained through the reverse operation).

Although it is simpler and faster than an individual locking system, also this multiple locking system has a number of structural and functional drawbacks:
a) it leaves a degree of freedom, because the L-shaped bracket can rotate upwards around the engagement rod since the coupling between them takes place between two curved surfaces, as is inevitable given the rotating movement of engagement, so that the nozzles could partially slip out of the irrigation ports;
b) since the rotation of the locking unit takes place around a fixed axis, it is necessary that all the L-shaped brackets are correctly positioned to be engaged by the engagement rod, therefore it is sufficient that only one bracket is out of position and the engagement rod will not be able to properly lock the whole row so that the operator will have to check all the brackets to understand which bracket is out of position (or the engagement rod can force the bracket against the instrument and damage it or damage the nozzles inserted in the bracket);
c) the rotation around the fixed axis also implies a geometric constraint on the position of the engagement rod and on the space for the rotation, which results in a greater encumbrance;
d) since there is also an axial displacement of the engagement rod to overcome the force of the end springs and engage/disengage the retaining notch when the engagement rod is already in contact with the ends of the brackets, this displacement tends by friction to generate a transverse force on the brackets and therefore on the nozzles, which can be damaged and/or damage the nozzle insertion seats in the irrigation ports and/or in the passage holes on the L-shaped bracket;
e) it works only with L-shaped brackets for inserting the nozzles into the irrigation ports on the front side of the instruments, but it is not suitable for instruments with irrigation ports on the top side.

The object of the present invention is therefore to provide a trolley that overcomes the above-described drawbacks of prior art trolleys. This object is achieved by means of a trolley as claimed in claim 1, which the locking system includes a locking unit rotating around an axis that moves in a plane parallel to the plane of the brackets and that follows a guide substantially shaped like a J, the locking unit including an engagement plate that performs an engagement movement that is a rototranslation with an almost parallel abutment on the brackets. Other advantageous features are recited in the dependent claims.

The main advantage of the trolley according to this invention is to maintain a simple and economical structure with a high level of user-friendliness, but with a locking system that guarantees a smaller encumbrance and better functionality. In fact, the aforementioned movement of the locking unit ensures a complete locking of the brackets by coupling two flat surfaces thus eliminating any degree of freedom, and since the engagement plate rests on the brackets from above even if a bracket is out of position it is automatically pushed into position and does not prevent the locking nor can it force the instrument onto the support. In addition, the structure of the locking system is much more compact and with greater design flexibility since the axis of rotation is not fixed and can be positioned depending on the exact shape of the locking unit.

A second advantage of the present trolley results from the absence of axial displacement of the locking unit, thus avoiding the risks highlighted above under d) as the nozzles are not stressed.

Still another advantage of this trolley is that it is able to receive instruments of different models with irrigation ports obtained both on the front side and on the top side and also in different positions.

These and other advantages and characteristics of the trolley according to the present invention will be evident to those skilled in the art from the following detailed description of an embodiment thereof with reference to the attached drawings in which:
Fig.1 is a front perspective view of a trolley according to the invention, with some elements removed for greater clarity, provided with support accessories for 12 robotic surgical instruments shown loaded on the trolley.
Fig.2 shows, by means of two series of partial front perspective views, the movement of the locking unit from the open/unlocked position (A), through an intermediate position (B), to the closed/locked position (C);
Fig.3 is a view analogous to the previous one but with the positions illustrated in lateral view and with an enlarged detail of the intermediate position (B); and
Fig.4 is a rear perspective view from the bottom of the locking system.

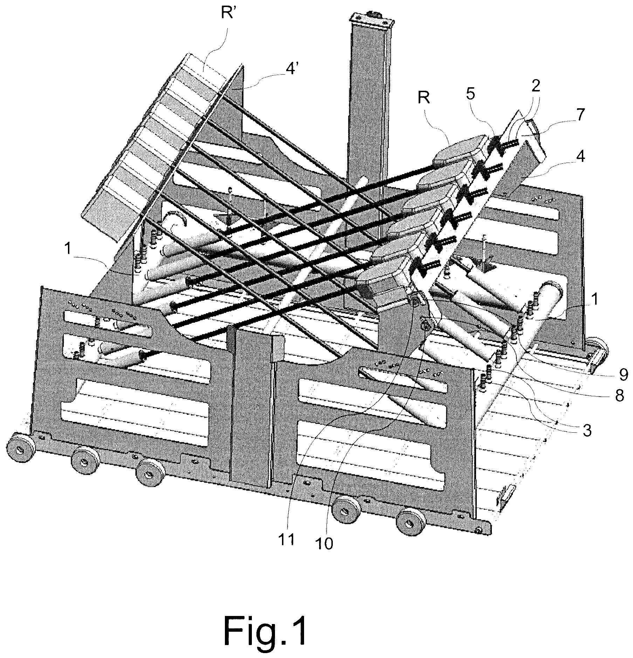

Referring to Fig.1, there is seen that a trolley for the washing and disinfection of robotic surgical instruments traditionally includes at least one horizontal cylindrical manifold 1 equipped with flexible washing tubes (not shown) provided with end nozzles 2 and mounted on respective couplings 3 that extend radially in a substantially vertical position from a series of outlets arranged along the manifold 1. It should be noted that couplings 3 are typically made in pairs (six pairs in the example shown) because each robotic surgical instrument has a primary irrigation port and a secondary irrigation port, a washing nozzle 2 being inserted into each of these irrigation ports.

The trolley preferably includes two parallel and opposing manifolds 1, as in the example shown, so that it is possible to load two sets of instruments R, R' also of different types, which are carried by specific supports 4, 4' each with its own instruments locking system (in Fig.1 only the locking system of support 4 is shown).

As mentioned before, each pair of nozzles 2 are inserted in through holes obtained in a bracket 5 before entering the irrigation ports of the instrument R which, in the example shown, are located on its top side. Note that if the irrigation ports were located on the front side of instruments R', as described above with reference to the prior art represented by trolley A 502 by Miele, brackets 5 would have an L-shape, but in this case a simple flat shape is sufficient and in both cases the operation of the present locking system does not change.

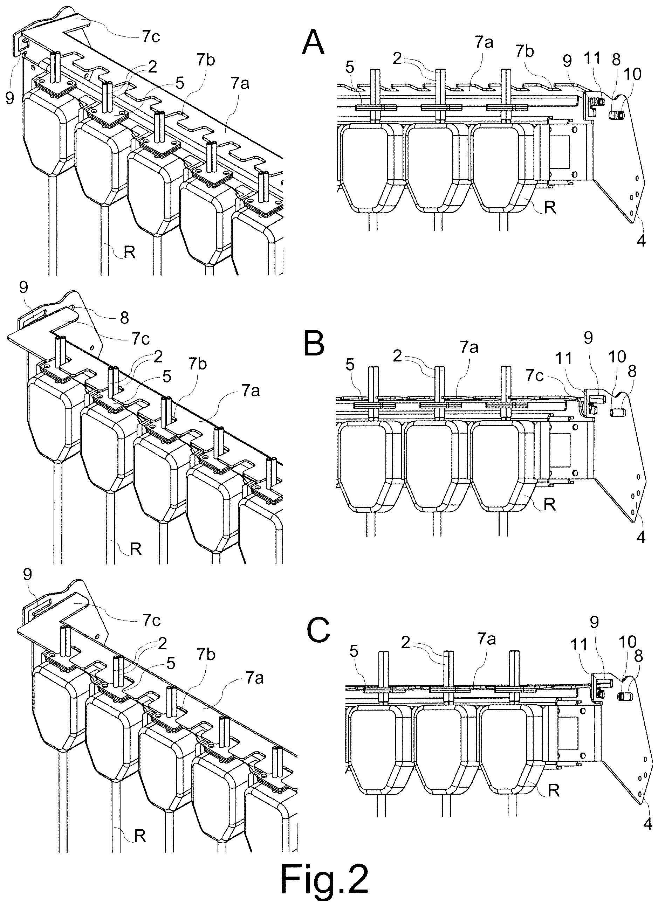

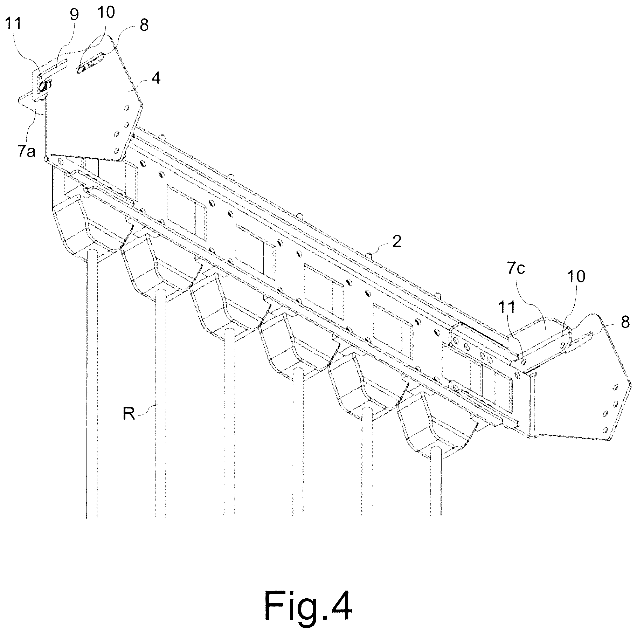

Referring now also to figures 2 to 4, there is seen that the main innovative aspect of the trolley according to the invention resides in the locking system which includes a locking unit 7 mounted on support 4 so that it can rotate and slide along a linear slot 8 and a guide 9 substantially shaped like a J. In practice, guide 9 is formed by a long side 9a and a short side 9b which are parallel and aligned at one end, and a transverse side 9c which connects them perpendicularly at said end which is the one closest to the seats of instruments R.

More specifically, both slot 8 and guide 9 are obtained in the ends of support 4 with slot 8 and sides 9a and 9b which extend in planes parallel or substantially parallel to plane P defined by brackets 5 when they are arranged at the top of instruments R and nozzles 2 are inserted in the irrigation ports. Slot 8 is slightly lower than plane P and guide 9 is slightly higher as far as its long side 9a is concerned, while its short side 9b is located just below plane P and above slot 8, and consequently its transverse side 9c is included in a plane T perpendicular to plane P and extends from above to below said plane P.

Locking unit 7 comprises an engagement plate 7a which is substantially comb-shaped, being provided with a plurality of recesses 7b obtained along the side facing nozzles 2, and is mounted on support 4 by means of end arms 7c extending outwards from the side opposite to recesses 7b. Each of the end arms 7c is provided on a side with a first pin 10 that engages slot 8 and a second pin 11 that engages guide 9.

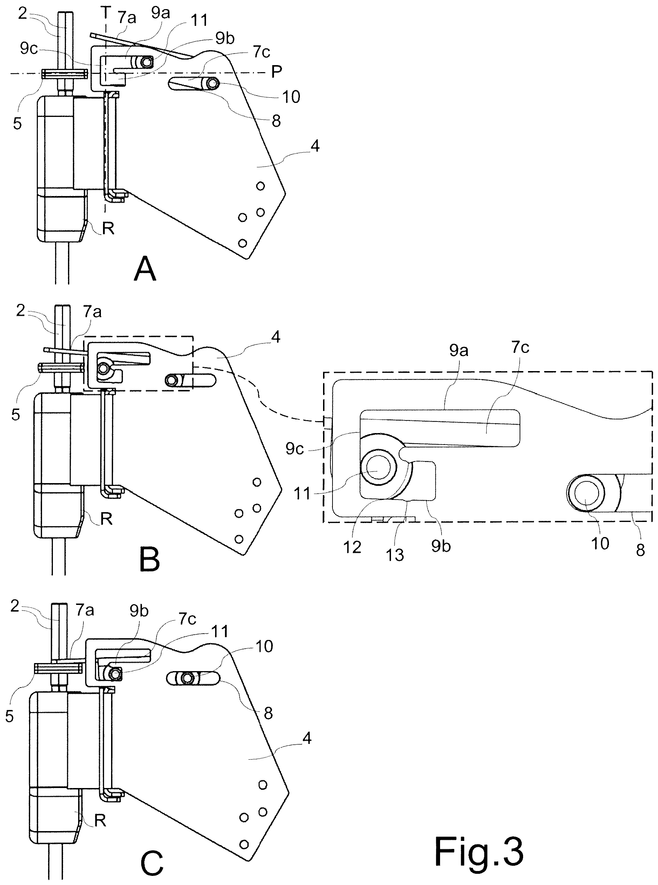

From the above description, the simple and effective operation of the locking system of the trolley according to the present invention is readily understood, illustrated in particular in figures 2 and 3.

The starting position A illustrates the system in the open/unlocked condition, in which the locking unit 7 is in the rearmost position so as not to interfere with the loading/unloading of the instruments R. In this position, the first pin 10 and the second pin 11 abut against the outer end of slot 8 and the long side 9a of guide 9 respectively, and the engagement plate 7a is inclined upwards with respect to plane P of brackets 5 in which nozzles 2 are inserted.

Each pair of nozzles 2 is arranged at a relevant recess 7b to avoid interference with the engagement plate 7a, which is preferably provided with a greater number of recesses 7b than brackets 5 so that it can also be used on other models of instruments with different positions of the irrigation ports. Note that if the locking system were only used for instruments R' with the irrigation ports on the front side, then not only would brackets 5 be L-shaped with the holes for nozzles 2 on the front side of the bracket, as mentioned above, but engagement plate 7a could be straight instead of comb-shaped since recesses 7b would not be necessary.

In the intermediate position B, the locking unit 7 was moved towards brackets 5 until the first pin 10 came into contact with the inner end of slot 8, and the second pin 11 entered transverse section 9c of guide 9 by slightly rotating the locking unit 7 downwards around the first pin 10. In this position, the engagement plate 7a extends almost completely over brackets 5 and nozzles 2 are accommodated in recesses 7b so as not to interfere with the movement of the locking unit 7.

The system then reaches the closed/locked condition of position C by bringing the second pin 11 into abutment on the outer end of the short side 9b, and the system preferably includes retention means to stably maintain this position. In the present embodiment said retention means are illustrated, in the enlarged detail of Fig.3, as an upper tooth 12 and a corresponding lower notch 13 formed in the short side 9b. The distance of these retention means from the outer end of the short side 9b is such as to keep the second pin 11 abutting against said end, and the overriding of the safety tooth 12 is made possible not only by the presence of the corresponding notch 13 but also by the same elasticity of the locking unit 7 which bends slightly.

In this position, the engagement plate 7a is in abutment on top of brackets 5 in an almost parallel condition and still extends beyond their centerline thus eliminating any degree of freedom since brackets 5, and consequently nozzles 2 and instruments R, are completely blocked. It should be noted that this position is achieved by a movement along guide 9 which combines an inwards translation along the long side 9a approaching brackets 5 (A→B), a rotation with descent in the transverse side 9c with almost parallel abutment onto the brackets (B), and an outwards translation along the short side 9b moving away for locking (B→C).

The unlocking is obviously done with a reverse movement that is just as simple, and in both movements there is no axial sliding of the locking unit 7 as it occurs in the prior art trolley model A 502 by Miele.

It is clear that the embodiment of the trolley according to the invention described and illustrated above is just an example susceptible to numerous variations, in addition to those already mentioned above. In particular, the exact shape and arrangement of the components can vary considerably depending on specific production requirements, as long as the general structure shown above is maintained. For example, brackets 5 may have a different shape and/or have a single hole if the washing of an instrument requires only one nozzle 2.

Similarly, recesses 7b may be absent, as mentioned above, or in a different number and/or with a different shape from that shown in the figures, possibly with an alternation of recesses of two different types in shape and/or size. Moreover, although the locking unit 7 is illustrated as a substantially flat structure in which the engagement plate 7a and the end arms 7c are coplanar, it would be possible to provide between them a positive or negative angle so that slot 8 would be consequently obtained higher or lower with respect to plane P.

Finally, in order to limit the size of the trolley in one direction rather than another, the locking system may be positioned differently in whole or in part with respect to the seats of instruments R, R'. For example, the ends of support 4 may extend inwards into the trolley so that slots 8 and guides 9 are located on the inside of the instrument seats, and as a result the end arms 7c would extend inwards instead of outwards. Or the entire locking system, whether in the version with slots 8 and guides 9 obtained inside or outside of the instrument seats, could be mirrored symmetrically on the inside of support 4, so that the initial locking movement of the second pins 11 along the long side 9a would also take place from the inside of the trolley to the outside.

## Claims

1. Trolley for an apparatus for the washing and disinfection of robotic surgical instruments (R), said trolley comprising at least one manifold (1) equipped with flexible washing tubes provided with end nozzles (2) and at least one support (4) with seats for said robotic surgical instruments (R) which is provided with a system for the simultaneous locking of all instruments (R) placed in said seats, said locking system comprising a series of brackets (5) equal in number to the instruments (R) and provided each with one or more through holes in which one or more of said end nozzles (2) are inserted before entering the irrigation port(s) of one of the instruments (R), as well as a locking unit (7) mounted in a rotatable way in seats formed in the ends of said support (4) and configured to engage said brackets (5) when they are placed at the top of the instruments (R) and the nozzles (2) are inserted in said irrigation port(s), **characterized in that** said locking unit (7) comprises an engagement plate (7a) which is mounted on the support (4) by means of end arms (7c) each provided on a side with a first pin (10) and a second pin (11) engaging respectively a linear slot (8) and a substantially J-shaped guide (9) formed in said ends of the support (4), both said linear slot (8) and a terminal short side (9b) of said guide (9) extending in planes parallel or substantially parallel to the plane (P) defined by the brackets (5) and spaced from it in such a way that said engagement plate (7a) is in abutment on the brackets (5) in an almost parallel condition and extends beyond their centerline when said second pins (11) are in abutment against the end of the guide (9) defined by said terminal short side (9b).

2. Trolley according to claim 1, **characterized in that** the engagement plate (7a) has a plurality of recesses (7b) formed along the side facing the nozzles (2), each recess (7b) being sized and positioned to accommodate at least one nozzle (2), preferably a pair of nozzles (2), so that the nozzles (2) do not come into contact with the engagement plate (7a) during the movement for the locking/unlocking of the brackets (5).

3. Trolley according to claim 2, **characterized in that** the number of recesses (7b) is higher than the number of brackets (5).

4. Trolley according to any of the preceding claims, **characterized in that** the engagement plate (7a), the linear slot (8) and the guide (9) are located in an external position with respect to the seats of the instruments (R).

5. Trolley according to any of the preceding claims, **characterized in that** the engagement plate (7a) is coplanar with the end arms (7c).

6. Trolley according to any of the preceding claims, **characterized in that** the locking system also includes retention means to stably maintain the locking position.

7. Trolley according to the previous claim, **characterized in that** the retention means consist of an upper tooth (12) and a corresponding lower notch (13) formed in the short side (9b) of the guide (9), the distance of said retention means from the end of the guide (9) being such as to keep the second pin (11) in abutment against said end.

8. Trolley according to any of the preceding claims, **characterized in that** it includes two parallel and opposite manifolds (1) and two supports (4, 4') for the instruments (R, R'), each of said supports (4, 4') being provided with its own system for the locking of the instruments (R, R').

9. Trolley according to the previous claim, **characterized in that** the two supports (4, 4') are different to receive different types of instruments (R, R'), and their relevant locking systems are in turn possibly different.

10. Trolley according to any of the preceding claims, **characterized in that** the linear slot (8) is located slightly lower than the plane (P) defined by the brackets (5) and the guide (9) is slightly higher as regards its long side (9a), while its short side (9b) is located just below said plane (P) and above the linear slot (8).

11. Apparatus for the washing and disinfection of medical items, **characterized in that** it includes a trolley according to any of the preceding claims.

## Patentansprüche

1. Aufnahmewagen für eine Vorrichtung zum Waschen und Desinfizieren chirurgischer Roboterinstrumente (R), wobei der besagte Aufnahmewagen wenigstens ein Verteilerrohr (1) umfasst, welches mit flexiblen Waschschläuchen mit endseitigen Düsen (2) eingerichtet ist, sowie wenigstens eine Stütze (4) mit Aufnahmen für die besagten chirurgischen Roboterinstrumente (R), die mit einem System zur gleichzeitigen Verriegelung aller in den besagten Aufnahmen platzierten Instrumente (R) versehen ist, wobei das besagte Verriegelungssystem eine Reihe von Halterungen (5) aufweist, welche von der gleichen Anzahl sind wie die Instrumente (R), und welche jeweils mit einem oder mehreren Durchgangslöchern versehen sind, in denen eine oder mehrere der besagten endseitigen Düsen (2) eingesetzt werden, bevor der (die) Spülanschluss (-anschlüsse) eines der Instrumente (R) eingesetzt wird (werden), sowie eine Verriegelungseinheit (7), die in drehbarer Weise innerhalb von in den Enden der besagten Stütze (4) eingeformten Aufnahmen gelagert ist und dazu ausgelegt ist, an den besagten Halterungen (5) einzurasten, wenn diese oben auf den Instrumenten (R) platziert werden und die Düsen (2) in dem (den) besagten Spülanschluss (-anschlüssen) eingesetzt sind, **dadurch gekennzeichnet, dass** die besagte Verriegelungseinheit (7) eine Einrastplatte (7a) umfasst, die an der Stütze (4) gelagert ist mittels endseitiger Arme (7c), von denen jeder an einer Seite vorgesehen ist, wobei ein erster Stift (10) und ein zweiter Stift (11) in einen linearen Schlitz (8) einrastet bzw. in eine im Allgemeinen J-förmige Führung (9), die in den besagten Enden der Stütze (4) eingeformt ist, wobei sowohl der besagte lineare Schlitz (8) als auch eine kurze Anschlussseite (9b) der besagten Führung (9) sich innerhalb von zu der von den Halterungen (5) definierten Ebene (P) parallelen oder substantiell parallelen Ebenen erstrecken, die davon derart weit entfernt sind, dass die besagte Einrastplatte (7a) an den Halterungen (5) in einem weitgehend parallelen Zustand anliegt und sich jenseits von deren Mittellinie erstreckt, wenn die besagten Stifte (11) an demjenigen Ende der Führung (9) anliegen, das durch die besagte kurze Anschlussseite (9b) definiert ist.

2. Aufnahmewagen gemäß Anspruch 1, **dadurch gekennzeichnet, dass** die Einrastplatte (7a) eine Mehrzahl von entlang der den Düsen (2) zugewandten Seite eingeformten Ausnehmungen (7b) aufweist, wobei jede Ausnehmung (7b) derart bemessen und positioniert ist, um wenigstens eine Düse (2) aufzunehmen, vorzugsweise ein Paar von Düsen (2), derart, dass die Düsen (2) während der Bewegung zur Verriegelung/Entriegelung der Halterungen (5) nicht in Kontakt mit der Einrastplatte (7a) gelangen.

3. Aufnahmewagen gemäß Anspruch 2, **dadurch gekennzeichnet, dass** die Anzahl der Ausnehmungen (7b) größer ist als die Anzahl der Halterungen (5).

4. Aufnahmewagen gemäß einem der vorangehenden Ansprüche, **dadurch gekennzeichnet, dass** die Einrastplatte (7a), der lineare Schlitz (8) und die Führung (9) an einer im Hinblick auf die Aufnahmen für die Instrumente (R) außen liegenden Position angeordnet sind.

5. Aufnahmewagen gemäß einem der vorangehenden Ansprüche, **dadurch gekennzeichnet, dass** die Einrastplatte (7a) in einer gemeinsamen Ebene mit den endseitigen Armen (7c) liegt.

6. Aufnahmewagen gemäß einem der vorangehenden Ansprüche, **dadurch gekennzeichnet, dass** das Verriegelungssystem auch ein Rückhaltemittel aufweist, um die Verriegelungsposition stabil einzuhalten.

7. Aufnahmewagen gemäß dem vorangehenden Anspruch, **dadurch gekennzeichnet, dass** das Rückhaltemittel aus einem oberen Zahn (12) sowie aus einer dazu passenden, in der kurzen Seite (9b) der Führung (9) ausgeformten, unteren Aussparung (13) besteht, wobei der Abstand des besagten Rückhaltemittels von dem Ende der Führung (9) derart ist, dass der zweite Stift (11) in Anlage gegen das besagte Ende gehalten wird.

8. Aufnahmewagen gemäß einem der vorangehenden Ansprüche, **dadurch gekennzeichnet, dass** er zwei parallele und einander gegenüber liegende Verteilerrohre (1) umfasst sowie zwei Stützen (4, 4') für die Instrumente (R, R'), wobei jede der besagten Stützen (4, 4') mit ihrem eigenen System zur Verriegelung der Instrumente (R, R') versehen ist.

9. Aufnahmewagen gemäß dem vorangehenden Anspruch, **dadurch gekennzeichnet, dass** die zwei Stützen (4, 4') unterschiedlich sind, um unterschiedliche Arten von Instrumenten (R, R') aufzunehmen, und dass ihre jeweiligen Verriegelungssysteme ihrerseits unterschiedlich sein können.

10. Aufnahmewagen gemäß einem der vorangehenden Ansprüche, **dadurch gekennzeichnet, dass** der lineare Schlitz (8) leicht unterhalb der von den Halterungen (5) definierten Ebene (P) liegt und die Führung (9) leicht oberhalb davon liegt, was ihre lange Seite (9a) betrifft, während ihre kurze Seite (9b) gerade unterhalb der besagten Ebene (P) liegt sowie höher als der lineare Schlitz (8).

11. Vorrichtung zum Waschen und Desinfizieren medizinischer Gegenstände, **dadurch gekennzeichnet, dass** sie einen Aufnahmewagen gemäß einem der vorangehenden Ansprüche umfasst.

## Revendications

1. Chariot pour un appareil pour le lavage et la désinfection d'instruments chirurgicaux robotisés (R), ledit chariot comprenant au moins un collecteur (1) équipé avec des tubes de lavage flexibles prévus avec des buses d'extrémité (2) et au moins un support (4) avec des sièges pour lesdits instruments chirurgicaux robotisés (R) qui est prévu avec un système pour le verrouillage simultané de tous les instruments (R) placés dans lesdits sièges, ledit système de verrouillage comprenant une série de consoles (5) égales en nombre aux instruments (R) et prévues chacune avec un ou plusieurs trous débouchants dans lesquels une ou plusieurs desdites buses d'extrémité (2) sont insérées avant d'entrer dans l'orifice (les orifices) d'irrigation de l'un des instruments (R), ainsi qu'une unité de verrouillage (7) montée d'une manière rotative dans les sièges formés dans les extrémités dudit support (4) et configurés pour mettre en prise lesdites consoles (5) lorsqu'elles sont placées au sommet des instruments (R) et les buses (2) sont insérées dans ledit (lesdits) orifice(s) d'irrigation, **caractérisé en ce que** ladite unité de verrouillage (7) comprend une plaque de mise en prise (7a) qui est montée sur le support (4) au moyen des bras d'extrémité (7c) chacun prévus sur un côté avec une première broche (10) et une seconde broche (11) mettant respectivement en prise une fente linéaire (8) et un guide sensiblement en forme de J (9) formé dans lesdites extrémités du support (4), à la fois ladite fente linéaire (8) et un côté court terminal (9b) dudit guide (9) s'étendant dans des plans parallèles ou sensiblement parallèles au plan (P) défini par les consoles (5) et espacés de ce dernier de sorte que ladite plaque de mise en prise (7a) est en butée sur les consoles (5) dans une condition presque parallèle et s'étend au-delà de leur ligne centrale lorsque lesdites secondes broches (11) sont en butée contre l'extrémité du guide (9) définie par ledit côté court terminal (9b).

2. Chariot selon la revendication 1, **caractérisé en ce que** la plaque de mise en prise (7a) a une pluralité d'évidements (7b) formés le long du côté faisant face aux buses (2), chaque évidement (7b) étant dimensionné et positionné pour loger au moins une buse (2), de préférence une paire de buses (2), de sorte que les buses (2) ne viennent pas en contact avec la plaque de mise en prise (7a) pendant le déplacement pour verrouiller / déverrouiller les consoles (5).

3. Chariot selon la revendication 2, **caractérisé en ce que** le nombre d'évidements (7b) est supérieur au nombre de consoles (5).

4. Chariot selon l'une quelconque des revendications précédentes, **caractérisé en ce que** la plaque de mise en prise (7a), la fente linéaire (8) et le guide (9) sont positionnés dans une position externe par rapport aux sièges des instruments (R).

5. Chariot selon l'une quelconque des revendications précédentes, **caractérisé en ce que** la plaque de mise en prise (7a) est coplanaire avec les bras d'extrémité (7c).

6. Chariot selon l'une quelconque des revendications précédentes, **caractérisé en ce que** le système de verrouillage comprend également des moyens de retenue pour maintenir, de manière stable, la position de verrouillage.

7. Chariot selon la revendication précédente, **caractérisé en ce que** les moyens de retenue se composent d'une dent supérieure (12) et d'une encoche inférieure (13) correspondante formée dans le côté court (9b) du guide (9), la distance desdits moyens de retenue par rapport à l'extrémité du guide (9) étant telle qu'elle maintient la seconde broche (11) en butée contre ladite extrémité.

8. Chariot selon l'une quelconque des revendications précédentes, **caractérisé en ce qu'**il comprend deux collecteurs parallèles et opposés (1) et deux supports (4, 4') pour les instruments (R, R'), chacun desdits supports (4, 4') étant prévu avec son propre système pour le verrouillage des instruments (R, R').

9. Chariot selon la revendication précédente, **caractérisé en ce que** les deux supports (4, 4') sont différents pour recevoir différents types d'instruments (R, R') et leurs systèmes de verrouillage en question sont à leur tour éventuellement différents.

10. Chariot selon l'une quelconque des revendications précédentes, **caractérisé en ce que** la fente linéaire (8) est positionnée légèrement plus bas que le plan (P) défini par les consoles (5) et le guide (9) est légèrement plus haut par rapport à son côté long (9a), alors que son côté court (9b) est positionné juste au-dessous dudit plan (P) et au-dessus de la fente linéaire (8).

11. Appareil pour le lavage et la désinfection d'accessoires médicaux, **caractérisé en ce qu'**il comprend un chariot selon l'une quelconque des revendications précédentes.
